# EUROPEAN PATENT APPLICATION

(11) **EP 2 796 556 A1**
(43) Date of publication of application: **29.10.2014**
(21) Application number: 13165387.5
(22) Date of filing: 25.04.2013
(51) Int. Cl.: C12N 15/67, A61K 38/16

(54) **Improved means and methods for expressing recombinant proteins**

(71) Applicant: Rijksuniversiteit Groningen, 9712 CP Groningen (NL)
(72) Inventor: Poolman, Berend, 9747 AG Groningen (NL); Linares-Martinez, Daniel, 9747 AG Groningen (NL); Gul, Nadia, 9747 AG Groningen (NL)
(74) Representative: Jansen, Cornelis Marinus

(57) **Abstract**

The invention relates to the field of genetic engineering and the production of recombinant proteins in microbial host cells. Provided is a method for enhanced expression of a recombinant protein of interest in a microbial host cell, comprising providing a microbial host cell wherein the function of H-NS (histone-like nucleoid structuring protein) or a homolog thereof is impaired; transforming the host cell with an expression vector comprising a nucleotide sequence encoding the recombinant protein; and culturing the transformed host cell in a culture media suitable for the expression of the recombinant protein.

## Description

The invention relates to the field of genetic engineering and the production of recombinant proteins in microbial host cells. More specifically, it relates to methods and means for enhanced expression of recombinant membrane proteins. It also relates to methods for identifying micro-organisms showing enhanced expression levels of recombinant proteins.

Microorganisms such as *Escherichia coli, Lactococcus lactis, Saccharomyces cerevisiae, Pichia pastoris* are generally preferred for the production of recombinant proteins. However, the heterologous expression of properly folded and functional proteins in these organisms remains very challenging. Membrane proteins are particularly difficult to handle and present additional difficulties during expression and purification.

Membrane proteins play a crucial role in a wide range of physiological processes including metabolic energy conservation, nutrient uptake, detoxification, regulation of cell volume and ion homeostasis, and signaling of environmental conditions. Membrane proteins represent 20-30% of the proteome of prokaryotic and eukaryotic organisms, and they comprise more than seventy percent of the current drug targets. However, our understanding of function-structure relationships of membrane proteins is low as compared to water-soluble proteins. This imbalance has been attributed to the unparalleled difficulties of obtaining sufficient amounts of protein for functional and structural studies. It has been noted that the most optimal host for overexpression is the native source (Bill et al., 2011; Grisshammer & Tate, 1995). However, this is not a feasible strategy for most membrane proteins since they are either not expressed at a high enough level or a suitable self-expression technology is not available (Shaw & Miroux, 2003).

The challenge of overproducing membrane proteins in a functionally competent state is enormous. The available literature suggest that many membrane proteins can be expressed in reasonable amounts, but often they fail to get inserted into the membrane in their native conformations and are non-functional (Georgiou & Valax, 1996; Klepsch, Persson, & de Gier, 2011). The difficulties to overproduce functional membrane proteins have been attributed to factors such as (i) toxicity of the proteins to the host cell (Laible, Scott, Henry, & Hanson, 2004); (ii) mismatch in codon usage and tRNA abundance (Zhang, Hubalewska, & Ignatova, 2009) (iii) limitation in the availability of precursors for protein synthesis (e.g. amino acids) (Marreddy et al., 2010); (iv) saturation of the membrane protein insertion machinery (Loll, 2003; Wagner, Bader, Drew, & de Gier, 2006); (v) insufficient chaperone and foldase activity (Higgins, Demir, & Tate, 2003; Tate, Whiteley, & Betenbaugh, 1999); and (vi) limited membrane space to accommodate extra protein (Arechaga et al., 2000).

Compared to soluble proteins, membrane proteins have a more complex biogenesis pathway and bottlenecks in their synthesis may arise from improper targeting, membrane insertion and/or folding (Georgiou & Valax, 1996; Klepsch et al., 2011). The level of correctly-folded membrane protein can often be enhanced by systematic optimization of several parameters such as (i) directed evolution or engineering of the expression host (Wagner et al., 2008) (ii) tuning of expression conditions such as temperature, growth medium and inducer strength (Marreddy et al., 2010) (iii) modification of target proteins (Wang et al., 2003; Zoonens & Miroux, 2010). Such screening is often done by trial-and-error and, hence, very time-consuming. More efficient and target-specific screening methods are needed to tackle the enormous expression space.

The present inventors recognized the need for new and improved heterologous protein expression systems. In particular, they aimed at providing molecular tools that allow to screen for functional expression and to monitor the correct folding of (membrane) proteins *in vivo.* Furthermore, they set out to identify optimized bacterial strains showing enhanced expression of recombinant (membrane) proteins.

To this end, they used an evolutionary strategy to optimize E. *coli* as host for the production of functional membrane protein. The folding reporter GFP was combined with the erythromycin-resistance protein (ErmC) to select for increased expression. Using different target proteins, it was surprisingly found that the generic transcriptional silencer H-NS is a factor that limits the overexpression of membrane proteins in wildtype *E*. *coli* cells. What is more, they observed that the degree of expression of (membrane) proteins correlates with the severity of the *hns* mutation. Thus, removal of the transcriptional silencing mechanism in a host cell is advantageously used to increase the level of functional overexpression of proteins.

Accordingly, the invention provides a method for enhanced expression of a recombinant protein of interest in a microbial host cell, comprising
- providing a microbial host cell wherein the function of H-NS (histone-like nucleoid structuring protein) or a functional analog thereof, is impaired;
- transforming the host cell with the expression vector comprising a nucleotide sequence encoding the recombinant protein; and
- culturing the transformed host cell in a culture media suitable for the expression of the recombinant protein.

The invention is characterized in that the transcriptional silencing mechanism of the host cell is at least partially impaired. This is effectively achieved by suppressing or abolishing the function of H-NS (histone-like nucleoid structuring protein) or a functional homolog thereof.

H-NS is an abundant nucleoid-associated protein (Dorman, 2004), involved in genome organization and transcriptional silencing of a variety of cellular functions. H-NS consists of N- and C-terminal domains separated by a flexible linker region. H-NS represses transcription by binding to high-affinity sites and cooperative spreading along DNA, thereby occupying the promoter region. As a consequence it also forms looped structures of DNA (Dame, Noom, & Wuite, 2006; Liu, Chen, Kenney, & Yan, 2010). In fact, H-NS and other nucleoid associated proteins (Fis, Dps and StpA) are thought to form large topological domain barriers, thereby determining the chromosome architecture. The interaction of H-NS is affected by several environmental factors, including pH, osmolarity, temperature and growth phase, but differs from gene to gene and as such the regulation of gene expression is complex.

A method of the invention involves culturing a host cell transformed with an expression vector comprising a nucleotide sequence encoding the recombinant protein in a culture media suitable for the expression of the recombinant protein, wherein the function of H-NS (histone-like nucleoid structuring protein) or a homologous gene in said host cell is suppressed. It has been shown in the art that the deletion or inactivation of a gene in a host cell can have an advantageous effect on recombinant gene expression.

US2012/183995 relates to Gram-positive microorganisms, such as Bacillus species having enhanced expression of a protein of interest, wherein one or more chromosomal genes have been inactivated. Preferred proteins of interest include enzymes, antibodies, hormones and growth factors. The method preferably comprises inactivating one or more chromosomal genes selected from the group consisting of *sbo, slr, ybcO, csn, spollSA, sigB, phrC, rapA, CssS trpA, trpB, trpC, trpD, trpE, trpF, tdh*/*kbl, alsD, sigD, prpC, gapB, pckA, fbp, rocA, ycgN, ycgM, rocF,* and *rocD.*

US2011/129487 discloses a mutated cell, such as a bacterium or yeast, in which the *thyA* gene coding thymidylate synthase includes a nonsense codon inducing the interruption of *thyA* gene translation and the auxotrophy of the cell for the thymidine. These cells are advantageously used as recombinant protein expression system. Preferably, the *endA* gene coding for endonuclease 1 and/or the *recA* gene coding for the recombinase are also inactivated in the mutated cell.

EP1173546 relates to a Bacillus cell for improved production of a polypeptide of interest by suppressing the expression of the *yjbH* gene or homolog thereof.

WO2012160027 relates to a host cell for the recombinant expression of a mammalian interferon protein, said cell comprising (a) a functional endogenous *trxB* gene; and (b) an inactivated endogenous glutathione oxidoreductase (*gor*) gene.

US 2005/0118689 relates to a process for the preparation of L-amino acids, in particular L-threonine in Enterobacteriaceae. It teaches that enhanced expression of, among others, the *H-NS* gene is advantageous for L-amino acid production.

Importantly however, none of the prior art studies teach or suggest to inactivate *H-NS* in order to improve recombinant protein expression.

According to the invention, suppression of H-NS function can be achieved by any suitable means, including chemical treatment and/or genetic modification of the microbial host cell. Preferably, it comprises genetic modification of the host cell e.g. inactivating the *hns* gene or a homologous gene such that the host cell expresses less than wild-type levels of the gene. Modification includes any alterations in a promotor and/or open reading frame region such as deletions, insertions, frameshifts or any manipulation of the DNA using methods known in the art.

Thus, in one embodiment of the invention, the host cell contemplated herein for the expression of recombinant protein has been genetically manipulated to inactivate the endogenous *hns* gene or homolog thereof, i.e. the gene encoding the H-NS protein or homolog thereof native to the host cell. The gene encoding the generic transcriptional silencer H-NS has been described in the literature in several different organisms. As an example, the nucleotide sequence of the *hns* gene of *E. coli* is provided herein as SEQ ID NO: 1. Based on the nucleotide sequence of the *hns* gene, the skilled person will readily be able to inactivate said gene by standard genetic methods, such as site-directed mutagenesis. The inactivation of the *hns* gene will be such that no or substantially no biologically active HNS protein is expressed in the host cell. For the purpose of the present invention, the *hns* gene will be considered as inactivated if the genetic manipulation has decreased the amount of functional H-NS (or homologous gene product) by at least 70%, preferably 85%, more preferably 95% or more compared to the unmodified host cell. This may refer to the expression level of H-NS protein or to other functional parameters such as the dimerization activity. For example, inactivation may involve one or more mutations in H-NS or homolog thereof which increase(s) the dissociation constant for self-association of H-NS monomers. As a result, less functional H-NS dimers will be formed *in vivo.*

As used herein, "homologous genes" refers to a pair of genes from different, but usually related species, which correspond to each other and which are identical or very similar to each other. The term encompasses genes that are separated by speciation (i.e., the development of new species) (e.g., orthologous genes), as well as genes that have been separated by genetic duplication (e.g., paralogous genes). As used herein, "ortholog" and "orthologous genes" refer to genes in different species that have evolved from a common ancestral gene (i.e., a homologous gene) by speciation. Typically, orthologs retain the same function in during the course of evolution. Identification of orthologs finds use in the reliable prediction of gene function in newly sequenced genomes.

Proteins involved in folding the chromosome are known as nucleoid-associated proteins (NAPs). Because of their DNA-binding ability, NAPs can also play an important role in global gene regulation. Each well-known NAP in Enterobacteriaceae may be categorized as a "factor for inversion stimulation" (Fis), "histone-like nucleoid structuring protein" (H-NS), "histone-like protein from Escherichia coli strain U93" (HU), "integration host factor" (IHF), or "leucine-responsive regulatory protein" (Lrp). Fis is one of the most abundant NAPs in exponentially growing *E. coli* cells, and its role as a transcriptional regulator has been investigated. H-NS binds DNA, especially A+T-rich regions including promoter regions or horizontally acquired DNA and acts as a global transcriptional repressor. HU and IHF are similar in amino acid sequence level, and both are global regulators, although they have distinct DNA-binding activities: HU binds to DNA nonspecifically whereas IHF binds to a consensus sequence. Lrp has a global influence on transcription regulation and is also involved in microbial virulence. In addition to these well-known NAPs, many other NAPs are found not only in Enterobacteriaceae but also in other organisms. For instance, NdpA, a functionally unknown NAP, has been found in Gram-negative bacteria. The MvaT family protein is the functional homolog of H-NS in *Pseudomonas* bacteria. The *Pseudomonas* MvaT/MvaU can complement phenotypes of *E. coli* hns mutants and selectively bind AT-rich sequences in a manner similar to H-NS.

Clearly identifiable H-NS homologs are found only in a subset of proteobacteria (Tendeng et al. (2003)Trends Microbiol 11:511-518). However, proteins lacking sequence similarity to H-NS may function as xenogeneic silencers in other bacteria. The Rok protein of *Bacillus subtilis* was also found to be a functional analog of H-NS. Lsr2 of *Mycobacterium tuberculosis* (Mtb) can complement hns phenotypes in *E. coli* and specifically binds and silences AT-rich regions of the mycobacterial genome. It was found that H-NS and Lsr2 use a common DNA binding mechanism where a short loop containing a "Q/RGR" motif selectively interacts with the DNA minor groove, where the highest affinity is for AT-rich sequences that lack A-tracts (Gordon et al., Proc Natl Acad Sci U S A. 2011 Jun 28;108(26):10690-5).

Other useful homologs of H-NS for practising the present invention include Hha and StpA. Hha is a small nucleoid-associated protein (8 kDa) involved in the negative modulation of virulence genes rather than of housekeeping genes in Gram-negative bacteria (Madrid et al. 2007. Mol. Microbiol. 63:7-14.30). Hha is a member of the Hha/YmoA family of proteins, which by themselves do not interact with DNA. StpA is an H-NS paralogue that is 58% similar to H-NS at the amino acid level. Due to this similarity, StpA was initially identified as a multi-copy gene suppressor that served as an H-NS substitute in H-NS-deficient cells. Both StpA and H-NS exhibit negative auto-regulation and are also able to suppress the promoter of the other.

Although Hha and StpA are much less abundant in the cell and play a less prominent role in gene regulation than H-NS, H-NS can form heterooligomeric complexes with these proteins, which increases the spectrum of gene targets (H-NS/StpA heteromers) or alter the repression at some loci (H-NS/Hha heteromers).

In one embodiment, suppression of H-NS function comprises inactivating in the host cell a gene comprising
(a) the DNA sequence shown in positions 1 to 414 in SEQ ID NO 1 (see below)
(b) a DNA sequence encoding an amino acid sequence shown in positions 1 to 137 in SEQ ID NO 2 (see below) or a homologous amino acid sequence.
(c) a DNA sequence which is at least 70%, preferably at least 80%, more preferably at least 90%, identical to the DNA sequence of item (a) or item (b).
SEQ ID NO:1 
1 atgagcgaagcacttaaaattctgaacaacatccgtactcttcgtgcgcaggcaagagaa
tgtacacttgaaacgctggaagaaatgctggaaaaattagaagttgttgttaacgaacgt
cgcgaagaagaaagcgcggctgctgctgaagttgaagagcgcactcgtaaactgcagcaa
tatcgcgaaatgctgatcgctgacggtattgacccgaacgaactgctgaatagccttgct
gccgttaaatctggcaccaaagctaaacgtgctcagcgtccggcaaaatatagctacgtt
gacgaaaacggcgaaactaaaacctggactggccaaggccgtactccagctgtaatcaaa
aaagcaatggatgagcaaggtaaatccctcgacgatttcctgatcaagcaataa 414
 SEQ ID NO:2

1 MSEALKILNNIRTLRAQARECTLETLEEMLEKLEVVVNERREEESAA
AAEVEERTRKLQQYREMLIADGIDPNELLNSLAAVKSGTKAKRAQRPAK
YSYVDENGETKTWTGQGRTPAVIKKAMDEQGKSLDDFLIKQ 137

"Homologous amino acid sequence" as used herein means a polypeptide sequence having 100%, 99%, 98%, 97%, 96%, 95%, 94%, 93%, 92%, 91%, 90%, 88%, 85%, 80%, 75%, or 70% sequence identity to the polypeptide sequence when optimally aligned for comparison. In some embodiments, homologous sequences have between 85% and 100% sequence identity, while in other embodiments there is between 90% and 100% sequence identity, and in more preferred embodiments, there is 95% or 97% sequence identity.

The term "inactivation" as used herein includes any method that prevents the functional expression of one or more of the *H-NS* or a homologous chromosomal genes, wherein the gene or gene product is unable to exert its known function. Inactivation occurs via any suitable means, including deletions, substitutions (e.g., mutations), interruptions, and/or insertions in the nucleic acid gene sequence. In one embodiment, the expression product of an inactivated gene is a truncated and/or mutated protein with a corresponding change in the biological activity of the protein. In a preferred embodiment, the inactivation of one or more *H-NS* genes results in stable and non-reverting inactivation.

Of particular interest is inactivation via alteration(s) in a specific domain of the H-NS protein or homolog thereof. For example, residues 1-60 of SEQ ID NO:2 represent the dimerization domain of H-NS of *E. coli* K-12. Residues 90-137 represent the DNA-binding domain of H-NS. In one embodiment, the host cell contains an alteration in the dimerization domain of H-NS which leads to a loss of activity. For example, one or more amino acids are substituted such that the dissociation constant is increased. In a specific aspect, the mutation is introduced in the region corresponding to amino acids 21-29 of *E. coli* H-NS. For example, one or more of Thr22 and Thr25 is replaced by a non-conserved residue, like Met. Other useful mutants include R15E, L26P, L30P and P115A that were previously shown to be unable to silence genes *in vivo* (see Lim et al. Sci Rep. 2012; 2: 509 and references cited therein).

In some preferred embodiments, inactivation is achieved by gene deletion. In some preferred embodiments, the gene is deleted by homologous recombination. For example, in some embodiments when the *H-NS* gene is to be deleted, a DNA construct comprising an incoming sequence having a selective marker flanked on each side by a homology box is used. The homology box comprises nucleotide sequences homologous to nucleic acids flanking regions of the chromosomal *H-NS* gene. The DNA construct aligns with the homologous sequences of the host chromosome and in a double crossover event the *H-NS* gene is excised out of the host chromosome. As used herein, "deletion" of a gene refers to deletion of the entire coding sequence, deletion of part of the coding sequence, or deletion of the coding sequence including flanking regions. The deletion may be partial as long as the sequences left in the chromosome provides the desired biological activity of the gene. The flanking regions of the coding sequence may include from about 1 bp to about 500 bp at the 5' and 3' ends. The flanking region may be larger than 500 bp but will preferably not include other genes in the region which may be inactivated or deleted according to the invention. The end result is that the deleted gene is effectively non-functional. In simple terms, a "deletion" is defined as a change in either nucleotide or amino acid sequence in which one or more nucleotides or amino acid residues, respectively, have been removed (i.e., are absent). Thus, a "deletion mutant" has fewer nucleotides or amino acids than the respective wild-type organism. In one embodiment, inactivation comprises deletion of the dimerization domain of H-NS or part thereof. For example, one or more of the nucleic acid sequence comprising or consisting of the nucleic acids 73-84 may be deleted, such that residues Thr25, Leu26, Glu27 and/or Glu28 in the dimerization domain are removed. In another embodiment, it comprises deletion of the DNA-binding domain of H-NS.

In another preferred embodiment, inactivation is by insertion. For example, in some embodiments, when the *H-NS* gene is to be inactivated, a DNA construct comprises an incoming sequence having the *H-NS* gene interrupted by a selective marker. The selective marker will be flanked on each side by sections of the *H-NS* coding sequence. The DNA construct aligns with essentially identical sequences of the *H-NS* gene in the host chromosome and in a double crossover event the *H-NS* gene is inactivated by the insertion of the selective marker. In simple terms, an "insertion" or "addition" is a change in a nucleotide or amino acid sequence which has resulted in the addition of one or more nucleotides or amino acid residues, respectively, as compared to the naturally occurring sequence.

In another embodiment, inactivation is by insertion in a single crossover event with a plasmid as the vector. For example, a *H-NS* chromosomal gene is aligned with a plasmid comprising the gene or part of the gene coding sequence and a selective marker. In some embodiments, the selective marker is located within the gene coding sequence or on a part of the plasmid separate from the gene. The vector is integrated into the host chromosome, and the gene is inactivated by the insertion of the vector in the coding sequence.

In yet alternative embodiments, inactivation results due to mutation of the gene. Methods of mutating genes are well known in the art and include but are not limited to site-directed mutation, generation of random mutations, and gapped-duplex approaches (See e.g., U.S. Pat. No. 4,760,025). As used herein, a "substitution" results from the replacement of one or more nucleotides or amino acids by different nucleotides or amino acids, respectively.

The host cell may have at least one further mutation which enhances the production of a recombinant (membrane) protein. Table 1 shows exemplary host cells comprising one or more mutations in addition to the functional disruption of H-NS.

For example, it has at least one further gene mutation in one or more genes selected from the group consisting of *ung, cpxA, dcm* and *ydcU,* and homologs thereof. In one embodiment, a mutation is present in the *ung* gene encoding uracil DNA glycosylase which modulates the mutation frequency and is controlled by the *cpx* regulon. For example, one or more residues in the region corresponding to amino acids 70-80 are mutated. In a specific aspect, the mutation is Gly74Ser. Alternatively or additionally, a mutation is present in the *cpxA* gene encoding histidine sensor kinase involved in cell envelope stress response. For example, one or more residues in the region corresponding to amino acids 170-180 are mutated. In a specific aspect, the mutation is Ser175Asn. In still a further aspect, a mutation is present in the *ydcU* gene encoding a putative ABC transporter for spermidine and putrescine.

In a preferred embodiment, functional disruption of H-NS is supplemented with a mutation in the *dcm* gene encoding DNA-cytosine methyltransferase. As an example, one or more residues in the region corresponding to amino acids 1420-1460, preferably 1430-1450, are mutated. In a specific aspect, the mutation is Val1446Glu. See for instance strain NG3 in Table 1.

A method of the invention is very suitable for large scale production of any protein of interest, be it of prokaryotic or eukaryotic origin. In a specific aspect, the protein of interest is a eukaryotic protein. As described herein above, the method is particularly advantageous for functional overexpression of a membrane protein, preferably a eukaryotic membrane protein, such as a transporter protein, a channel protein or a receptor protein. In a specific embodiment, it is a G-protein coupled receptor, such as the PDGF receptor, or the pore-forming integral membrane protein aquaporin.

The invention may be practiced using any microbial host cell. For large-scale production of recombinant proteins, bacterial and fungal expression hosts such as *Escherichia coli, Lactococcus lactis, Saccharomyces cerevisiae* and *Pichia pastoris* are preferred. In view of the ease of handling and the rapid cell growth, the use of bacterial host cells is particularly preferred herein. Bacterial cells from numerous species have been used in the art for the expression of heterologous proteins. Commonly used host cells comprise strains of *Escherichia coli, Lactococcus lactis* and *Bacillus subtilis.* Although every organism has its pros and cons for the production of complex (multi-domain) membrane proteins, *E. coli* remains the most widely used host for the heterologous expression of proteins in high quantities (Mergulhao, Summers, & Monteiro, 2005; Raman, Cherezov, & Caffrey, 2006; Yin, Li, Ren, & Herrler, 2007). The genetics and the fundamental understanding of transcription, translation and protein folding are far better characterized than for any other (micro)organism. In a preferred embodiment, the bacterial host cell is an *E. coli* cell, such as a cell that has been derived from *E. coli* strain MG1655 or MC1061.

According to the invention, the host cell having impaired function of H-NS or homolog thereof is transformed with an expression vector comprising a nucleotide sequence encoding the recombinant protein of interest. Also provided herein is the use of a microbial cell wherein the function of H-NS (histone-like nucleoid structuring protein) or a homolog thereof is impaired as host cell for expression of a recombinant protein, preferably a membrane protein. A still further aspect relates to a microbial host cell wherein the function of H-NS (histone-like nucleoid structuring protein) or a homolog thereof is impaired, the host cell comprising an expression vector with gene(s) encoding a (heterologous) recombinant protein, preferably a membrane protein.

Expression vectors are known in the art and typically comprise a replication origin, a nucleotide sequence coding for a recombinant protein of interest, and an expression cassette for expression of said recombinant protein of interest in the host cell. The vector may include other additional features besides the transgene insert and a backbone, such as promoter, a multiple cloning site, genetic marker(s), antibiotic resistance gene(s), reporter gene(s), protein targeting sequence, protein purification tag.

A promoter is used to drive transcription of the vector's transgene. A multiple cloning site allows for insertion of foreign DNA. Vectors with antibiotic-resistance open reading frames allow for survival of host cells that have taken up the vector in growth media containing antibiotics through antibiotic selection. The expression vector may contain a reporter gene that allow for identification of plasmid that contains inserted DNA sequence. Other commonly used reporters include green fluorescent protein and luciferase.

Expression vectors may include a nucleotide sequence encoding for a targeting sequence in the finished protein that directs the expressed protein to a specific organelle in the cell or specific location, such as the periplasmic space of bacteria. Expression vectors may include protein or peptide sequences that allow for easier purification of the expressed protein of interest. Examples include polyhistidine-tag, glutathione-S-transferase, and maltose binding protein. Some of these tags may also allow for increased solubility of the target protein. The target protein is fused to the protein tag, but a protease cleavage site positioned in the polypeptide linker region between the protein and the tag allows the tag to be removed later.

If the host cell is a bacterial cell, such as an *E. coli* cell, the expression vector will be adapted to protein expression in a prokaryotic cell environment. A vast number of expression vectors have been described for *E. coli* and other bacterial hosts. Examples for vectors suitable for protein expression in *E. coli* cells comprise, for example, the vectors of the pBluescript series, the vectors of the pUC series or a vector that belongs to the pET group of vectors. pET vectors typically comprise a *lacI* gene which codes for the lac repressor protein, a T7 promoter which is specific to T7 RNA polymerase, a transcription termination sequence, a lac operator which can block transcription, a polylinker for cloning, an f1 origin of replication, an ampicillin or kanamycin resistance gene, and a ColE1 origin of replication. pET vectors for use in the methods of the present invention comprise pET-21a(+), pET-24a(+), pET-28a(+), pET-29a(+), pET-30a(+), pET-41a(+), pET-44a(+), pET-21b(+), pET-24b(+), pET-26b(+), pET-28b(+), pET-29b(+), pET-30b(+), pET-42b(+),pET-44b(+). Vectors which are based on the pET-26b(+) backbone are particularly preferred. Further examples of suitable vectors are described, e.g. in " Cloning Vectors" (Pouwels et al. (eds.) Elsevier, Amsterdam New York Oxford, 1985).

The expression of membrane proteins is often harmful to the host cell,and manifested as inhibition of growth. This complicates gene cloning and protein production. Therefore, vectors with tight and controllable promoter systems are preferred. Since the amount of functional protein is generally more important than the total amount of protein, plasmid vectors with a moderate copy number and tunable promoter systems are preferred. Also, it is advantageous to use a plasmid vector in which the gene of interest can be fused in frame with the gene encoding GFP, which can be used as folding reporter for the protein of interest (as described in Geertsma et al.,2008).

Of particular interest for inducing target protein expression in a method of the invention is a series of plasmid vectors (pBAD vectors) containing the PBAD promoter of the araBAD (L-arabinose) operon and the gene encoding the positive and negative regulator of this promoter, araC. Using the phoA gene and phoA fusions to monitor expression in these vectors, it was shown that the ratio of induction/repression can be 1,200-fold, compared with 50-fold for PTAC-based vectors (Guzman et al., J Bacteriol. 1995 Jul;177(14):4121-30). Using a pBAD vector, target protein expression can be modulated over a wide range of inducer (arabinose) concentrations and reduced to extremely low levels by the presence of glucose, which represses expression. Also, the kinetics of induction and repression are very rapid and significantly affected by the ara allele in the host strain. Thus, the use of this system which can be efficiently and rapidly turned on and off allows the study of important aspects of bacterial physiology in a very simple manner and without changes of temperature. Modulation of protein expression can be of high relevance for expression of a membrane protein in a host cell.

In a further aspect, the invention provides a microbial host cell wherein the function of H-NS or a homolog thereof is impaired, and wherein the host cell comprises an expression vector with gene(s) encoding a (heterologous or homologous) recombinant protein of interest, preferably a membrane protein. For example, the host cell is an *E. coli* cell comprising an expression vector wherein the gene is under the control of an inducible *E. coli* operon. Preferably, the inducible operon is the L-arabinose operon containing the P_{BAD} promoter of the araBAD operon.

When cloning a polynucleotide sequence which encodes a eukaryotic (membrane) protein of interest into a bacterial expression vector, it should be considered that it may be helpful to modify the polynucleotide sequence in order to adapt it to prokaryotic expression. Methods for optimizing the codon-usage of a polynucleotide sequence to be expressed to render it amenable for expression in bacterial host cells, such as *E. coli,* are well known in the art. Briefly, the polynucleotide sequence which encodes the protein to be expressed in the host cells is manipulated by adapting the codon usage to the codon bias of *E. coli.* Regions of very high (> 80%) and very low (< 30%) GC content were avoided where possible. Also, cis-acting sequence motifs, such as internal TATA-boxes, chi-sites and ribosomal entry sites, repeated sequences, RNA secondary structures and AT-rich or GC-rich sequence stretches were avoided. The optimization of gene sequences, such as human gene sequences, for expression in bacterial host cells like *E. coli* is also carried out by commercial providers like Geneart (Regensburg, Germany).

An expression vector for use in bacterial cells may be transformed into the host cell by any suitable method. For example, an expression vector for use in *E. coli* may be introduced into the host cell, e.g. by electroporation or by chemical methods such as calcium phosphate-mediated transformation, as described in Maniatis et al. 1982, Molecular Cloning, A laboratory Manual, Cold Spring Harbor Laboratory.

The transformed host cell is then cultured in a media suitable for the expression of the recombinant protein using standard techniques. The specific conditions will depend on the choice of the specific expression system and will also depend on factors like the nature of the host cell, the nature of the inducible promoter, and the like. The skilled person will readily be able to select and optimize the conditions which are required for the effective expression of the protein of interest in the host cell culture without any inventive effort.

For example, culturing of *E*. *coli* host cells having impaired H-NS function and expressing a protein of interest can be performed in accordance with standard fermentation methods. Typically, *E. coli* cells can be cultured in a batch or fed-batch process. Culturing methods for bacterial host cells are generally described in Encyclopedia of Bioprocess Technology: Fermentation, Biocatalysis, and Bio-separation, Volumes 1-5, Flickinger, M.C., Drew, S.W. (eds.), 1999 John Wiley & Sons. Typical temperature conditions will be in the range of 20°C to 42°C, more commonly 30°C to 40°C, but for the functional expression of membrane proteins a reduced temperature of 20°C to 25°C is often preferred. The culture medium will typically have a pH of between 6.5 and 9.0, more typically 7.0 to 8.0, e.g. 7.5. Fermentation of the culture will be continued for a time period ranging from several hours to several days. In particular, if the culturing is performed as a batch process, the culturing time normally ranges from about 12 hours to about 36 hours.

As a last step of the method, the (membrane) protein of interest may be obtained from the host cell. In case of a soluble protein, the protein may be isolated from the cytosol of the host cells. The cells can be disrupted, e.g., by a French press, by repeating cycles of freezing and thawing, or by sonication. Cellular debris can be easily removed from the protein fraction by centrifugation. The soluble protein can then be purified from other protein and non-protein components by standard chromatography methods, including, for example, size exclusion chromatography and/or ion exchange chromatography. Other methods which are typically applied in protein purification, such as ammonium sulphate precipitation and filtration and/or dialysis may also be used. The protein expressed by the host cell of the invention may also be purified by affinity purification using, e.g., monoclonal or polyclonal anti-interferon antibodies.

Membrane proteins are classified based on the level of interaction with membrane lipid bilayers, with peripheral membrane proteins associating non-covalently with the membrane, and integral membrane proteins associating more strongly by means of hydrophobic interactions. Generally speaking, peripheral membrane proteins can be purified by milder techniques than integral membrane proteins, whose extraction requires phospholipid bilayer disruption by detergents. Here, important criteria for strategies of membrane protein purification are addressed, with a focus on the initial stages of membrane protein solubilisation, where problems are most frequently encountered. Protocols are available in the art for the successful extraction of peripheral membrane proteins, solubilisation of integral membrane proteins, and detergent removal which is important not only for retaining native protein stability and biological functions, but also for the efficiency of later purification techniques. See for example Smith ("Strategies for the purification of membrane proteins", Methods Mol Biol. 2011;681:485-96).

To simplify purification, the recombinant protein produced according to the method of the invention may be expressed as a fusion protein which comprises an affinity tag that facilitates binding of the fusion protein via a compound exhibiting binding affinity to the tag. For example, the affinity tag may be a polyhistidine tag comprising 6-12 histidine residues which specifically interacts with a nickel ion chelate matrix. Alternatively, the tag may be glutathione-S-transferase allowing the purification on a glutathione matrix. Further affinity tags are well-known in the art. Non-limiting examples for pairs of affinity tag and affinity ligand include maltose-binding-protein (MBP) and maltose; avidin and biotin; Streptag and streptavidin; myc epitope and antibody. Where the affinity tag is a peptide or polypeptide, such tag may conveniently be expressed together with the target protein as a single expression product. Where the affinity tag is not of proteinaceous origin, it may be attached by chemical coupling reactions. For example, biotin may be chemically coupled to the fusion protein.

Instead of isolation of the expressed target protein from the host cell, the protein may be used for further analysis while being part of the host cell. For example, a microbial host cell expressing a membrane protein is suitably used for functional screening of membrane protein properties and/or of compounds capable of modulating the membrane protein. When a specific ligand is available for the protein of interest, drug screening on functionally overexpressed protein can be done without to purify the protein. Thus, the invention also provides a method for a method for analyzing at least one property of a recombinant membrane protein of interest expressed by a microbial host cell, comprising
- providing a microbial host cell wherein the function of H-NS (histone-like nucleoid structuring protein) or a homolog thereof is impaired;
- transforming the host cell with an expression vector comprising a nucleotide sequence encoding the membrane protein;
- culturing the transformed host cell in a culture media suitable for the expression of the recombinant membrane protein; and
- subjecting the host cell to the analysis of the at least one property of the membrane protein. Preferably, the property is selected from ligand binding affinity, signal transduction, transport activity, channel activity or methods to probe structural homogeneity (e.g. size-exclusion chromatography coupled to light-scattering. In one embodiment, a host cell expressing a G-protein coupled receptors (GPCR), transporter or channel protein is analyzed to identify novel drug candidates.

Another aspect of the invention relates to a method to screen for novel host cells having an improved capacity to express a functional and properly folded target protein, preferably a membrane protein. Up till to date, the production of integral membrane proteins in a fully functional state still is a hit-or-miss affair. Ideally, one would like to tackle the expression problem without having to rely on optimization without trial-and-error.

The present inventors set out to develop a simple and effective strategy to optimize the level of functional (membrane) protein expression. The approach is based on a double translational fusion reporting system, using the *gfp* and *ermC* genes in tandem and fused 3' of the target gene. The GFP protein is used as reporter to monitor the folding state of the target protein; the ErmC protein (23S rRNA adenine N-6 methyl-transferase) is used to screen for increased expression by evolving strains towards increasing concentrations of erythromycin. A manageable screen for expression optimization can be performed in as little as 200 µl of culture and done for numerous target proteins in parallel. Whereas the novel strategy is exemplified to evolve *E. coli* for enhanced production of integral membrane proteins, the method is applicable for any type of protein. When the technology is used in its full potential, it can provide a panel of expression hosts, each evolved and optimized for a given class of proteins or tuned towards a specific protein.

Thus, in a further embodiment of the invention provides a method to identify a microbial host cell having an improved capacity to express a functional and properly folded target protein, the method comprising the steps of :
(a) providing a population of microbial host cells transformed with an expression vector comprising a target nucleic acid sequence encoding the target protein, wherein the target nucleic acid sequence is fused C-terminally with a protein folding reporter construct and a selectable marker which confers a drug resistant phenotype when located in the cytoplasm;
(b) inducing expression of the proteins encoded by the expression vector
(c) serial culturing the transformed host cells at gradually increasing antibiotic concentrations;
(d) selecting the host cells showing increased expression of the reporter construct and,
(d) optionally, analyzing the genotype of the selected host cells.

It has been previously shown that one can screen for expression of membrane proteins in an overall well-folded state by fusion of a fluorescent reporter to the C-terminus of the target protein. If the target protein folds correctly the reporter is likely to reach its native conformation and become fluorescent (Waldo, Standish, Berendzen, & Terwilliger, 1999). A target protein that misfolds and aggregates will interfere with the folding of the reporter and be non-fluorescent. Thus, by monitoring the fluorescence of the reporter protein, one obtains a first indication of the folding state of the target protein. Earlier work has shown a very good correlation between fluorescence and the amount of membrane protein that is stably inserted into the membrane and functional (Drew, Lerch, Kunji, Slotboom, & de Gier, 2006; Drew et al., 2008; Geertsma, Groeneveld, Slotboom, & Poolman, 2008). Preferably, the protein folding reporter construct encodes a fluorescent protein, preferably GFP or derivative.

It has been established that GFP can serve as folding reporter and quantitatively assess the functionality of membrane proteins. In brief, when a protein, fused N-terminal to GFP, becomes misfolded during the biosynthesis, it turns GFP into a mis-folded, SDS-sensitive state. If the protein becomes properly folded, the GFP ß-barrel will be assembled as a fluorescent SDS-resistant moiety; the SDS-sensitive and SDS-resistant conformations can be readily discriminated on SDS-PAGE and immunoblots. Whole-cell and in-gel fluorescence of protein extracts have been shown to match the activity of membrane proteins such as the lactose transporters from *Streptococcus thermophilus* (LacSΔIIA) and E. *coli* (LacY) and the glutamate transporter (GltP) from *E. coli* ((Drew et al., 2006; Geertsma et al., 2008).

The target (membrane) protein is fused to a C-terminal selectable marker that confers a drug resistance phenotype, when located in the cytoplasm. In the initial studies, the inventors screened various antibiotic resistance markers, including the chloramphenicol (CAT), the tetracycline (TetA) and the kanamycin (Aph) resistance proteins as fusion partners for selection, but none of these markers proved useful in *E. coli.* Either the fusion partners were degraded or they dragged the target protein into a misfolded state. Because each of these antibiotic resistance proteins form an oligomeric complex (CAT and TetA are trimeric proteins and Aph is homodimeric), it was reasoned that their quaternary structure and/or cellular location (TetA is a membrane protein) posed a problem, in particular when the membrane protein target would form an oligomer as well. They then identified the erythromycin-resistance protein (ErmC) as a monomeric, soluble antibiotic resistance protein (Linares et al, 2010). Fusions of membrane proteins with ErmC proved to be highly stable. Thus, if a screening method of the invention involves the use of *E. coli* the selectable marker preferably confers resistance against erythromycin.

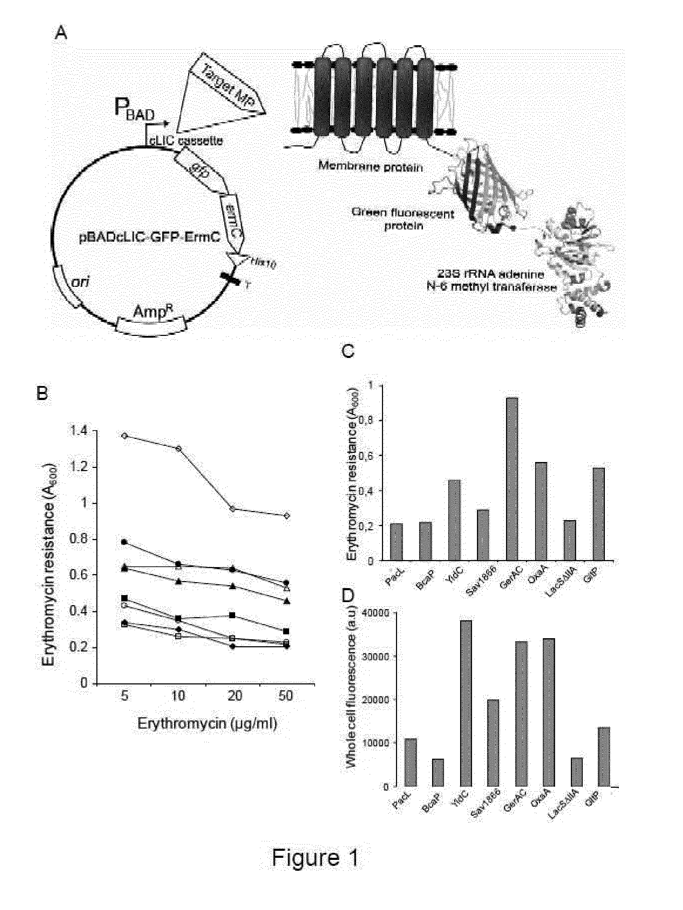

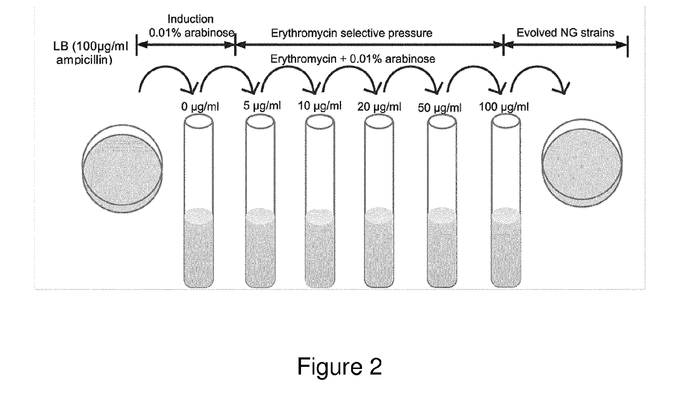

A method of the invention can be practised using any type of expression host, provided that the wild-type host (i.e. not expressing the double translational fusion reporting system) displays a sufficient level of sensitivity for the drug. For example, if erythromycin is used to select evolved hosts, the first requirement for the suitability of ErmC-fusions is a high sensitivity of the expression host for erythromycin. As many *E. coli* strains are relatively insensitive to erythromycin, we evaluated the isogenic *E. coli* strains BW25113, *E. coli* BW25113A (Δ*acrB*) and BW25113B (Δ*acrB* Δ*emrE mdfA*::kan) (Tal & Schuldiner, 2009). The differences in growth in erythromycin-containing media were marked. *E. coli* BW25113 could grow up to 100 µg/ml of erythromycin, albeit that the final yield was reduced. By comparison, *E. coli* BW25113B was completely inhibited at 10 µg/ml erythromycin, whereas *E. coli* BW25113A displayed an intermediate phenotype. Thus, in one embodiment the invention provides a method to identify a microbial host cell having an improved capacity to express a functional and properly folded protein of interest, preferably a membrane protein, the method comprising the steps of (a) providing a population of microbial host cells, wherein the host cell is *E. coli* strain BW25113A (Δ*acrB*) or BW25113B (Δ*acrB ΔemrE ΔmdfA:kan*) transformed with an expression vector comprising a target nucleic acid sequence encoding the target protein, wherein the target nucleic acid sequence is fused C-terminally with a protein folding reporter construct and the selectable marker ErmC which confers resistance to erythromycin (see e.g. Fig. 1); (b) inducing expression of the proteins encoded by the expression vector (c) serial culturing the transformed host cells at gradually increasing erythromycin concentrations;(d) selecting the host cells showing increased expression of the reporter construct and, (e) optionally, analyzing the genotype of the selected host cells. See Figure 2 for a schematic representation. Exemplary host cells that can be obtained using such method include the evolved NG strains indicated in Table 1 herein below. E. *coli* strain BW25113B is a preferred expression host.

MP-GFP-ErmC fusions were made with LacSΔIIA, BcaP, GltP, Sav1866, YidC and OxaA as target membrane protein. We observed two differently migrating protein species on immunoblots, the lower one being fluorescent in the in-gel assay (data not shown). This is consistent with previous observations on single fusions and show that a fraction of the target protein is well-folded and another fraction is misfolded. Thus, GFP can be used as reporter of the folding state of membrane proteins in dual fusions with ErmC. Fusions of GFP-ErmC were also made with the water-soluble proteins GerAC (spore germination factor) and LmoSBP (substrate-binding protein).

### LEGEND TO THE FIGURES

### Figure 1

Panel A: Plasmid-based selection system. The coding sequence of the target membrane protein is N-terminally fused in tandem to the folding reporter GFP and to a selectable marker encoding the 23S-rRNA adenine N-6-methyltransferase (ErmC). The expression cassette combines the *P_{BAD}* promoter with a high-throughput (ligation independent) cloning system.

Panel B: Survival at different concentrations of erythromycin. *E*. *coli* BW25113B cells overexpressing membrane proteins as GFP-ErmC fusions; the membrane proteins are (◆) PacL, (▲) YidC, (■) Sav1866, (○) LacSΔIIA, (●) OxaA, (0) GerAC, (□) BcaP and (Δ) GltP. Cells were grown at 20 °C on selective media with 5, 10, 20 or 50 µg/ml erythromycin and in the presence of 0.001% L-arabinose. No growth was observed in uninduced cultures, not even at the lowest concentration (2.5 µg/ml) of erythromycin tested.

Panel C. For each protein, the final OD₆₀₀ of the culture at an erythromycin concentration of 50 µg/ml is given. Panel D, like panel C but now the whole cell fluorescence is plotted.

### Figure 2

Scheme of the selection procedure to obtain a host cell with enhanced protein expression.

### Figure 3

Panel A: Selection of evolved strains. The in-gel GFP fluorescence and immunoblot data show the increase in LacSΔIIA expression with increasing erythromycin concentration. Samples were normalized based on OD₆₀₀; 25 µg total protein loaded/lane. Panel B: Comparison of best expressing clones (black) with parental strain BW25113B (grey); the NG strains were transformed with the same plasmid as used for evolving the strain: pBADc Lic-Gltp-GFP-ErmC in case of NG2 and NG3, pBADc Lic-Bcap-GFP-ErmC in case of NG5 and NG6.

### Figure 4

Screening for optimal inducer (L-arabinose) concentration of the expression of the glutamate transport protein (GltP). Panel A: Two representative strains, NG3 (■) and NG5 (A), are compared with the parental strain BW25113B (●). Panel B: In-gel GFP fluorescence and immunoblots probed with an anti-His tag antibody of the same gels are shown. Black and white arrows indicate the positions of non-fluorescent (misfolded) and fluorescent (folded) protein species, respectively.

### Figure 5

Panel A: Screening for optimal induction time at an L-arabinose concentration of 0.01% (w/v) of the expression of GltP. Samples were taken after 4 h (grey) and 16h (black) of induction. Panel B: Comparison of expression of GltP in BW25113B, NG2 and NG3 at 0.01% (w/v) L-arabinose and different induction times. In-gel GFP fluorescence and immunoblots probed with an anti-His tag antibody of the same gels are shown. Black and white arrows indicate the positions of non-fluorescent (mis-folded) and fluorescent (folded) protein species, respectively.

### Figure 6

Three representative membrane proteins expressed in the NG strains. L-arabinose at 0.01% (w/v) was used for overnight induction. In-gel GFP fluorescence and immunoblots probed with an anti-His tag antibody of the same gels are shown. Black and white arrows indicate the positions of non-fluorescent (mis-folded) and fluorescent (folded) protein species, respectively. (Panel A) BcaP-GFP-ErmC, (Panel B) GltP-GFP-ErmC, (Panel C) LacSΔIIA-GFP-ErmC (Panel D) LacSΔIIA-GFP. One of the proteins (LacSΔIIA) was expressed with (panel C) and without ErmC (panel D) as C-terminal fusion.

### EXPERIMENTAL SECTION

### MATERIALS AND METHODS

### Strains and cultivation conditions for protein expression

For cloning procedures *E. coli* MC1061 (Casadaban & Cohen, 1980) was used as host, and *E. coli* BW25113 Δ*acrB* Δ*emrE mdfA*::kan (Tal & Schuldiner, 2009; hereafter the strain is referred as BW25113B) was used as host for the selection of strains with improved (evolved) expression performance. *E. coli* strains carrying pBADcLIC derivatives were cultivated under vigorous aeration at 37 °C in Luria broth supplemented with 100 µg/ml ampicillin. In the case of *E. coli* BW25113 and derivatives, Luria broth was supplemented with 100 µg/ml ampicillin plus 5 µg/ml kanamycin. Cells were grown in Luria broth containing 100 µg/ml ampicillin *w*/*wo* 5 µg/ml kanamycin at 37°C in shake-flasks rotated at 200 rpm.

### Selection and screening

*E. coli* BW25113B transformed with pBADc LacSΔIIA-GFP-ErmC, pBADc Gltp-GFP-ErmC and pBADcBcap-GFP-ErmC were grown in LB supplemented with 100 µg/ml ampicillin plus 5 µg/ml kanamycin. The temperature was lowered from 37°C to 25°C after the A₆₀₀ had reached 0.5-0.6, and, after temperature equilibration, protein expression was induced by the addition of 0.01% (w/v) of L-arabinose. After 2 h of induction, a 2% (v/v) inoculum was introduced into fresh LB supplemented with 100µg/ml ampicillin, 5 µg/ml kanamycin plus 5 µg/ml erythromycin. Cultivation was continued for 48 h, after which the cells were diluted into fresh medium with an elevated erythromycin concentration (always using a 2% v/v culture inoculum). The erythromycin concentration was gradually increased from 5 to 10 to 20 to 50 to 100 to 200 µg/ml. After that cells were plated onto LB agar supplemented with 100 µg/ml ampicillin, 5 µg/ml kanamycin, 100 µg/ml erythromycin plus 0.01% w/v L-arabinose. Isolated colonies were selected for further analysis.

### Construction of GFP-ErmC fusion proteins

DNA manipulations were done according to standard protocols. The vector pBADcLIC-ERY was obtained by insertion of the cLIC-ERY cassette from pREcLIC-ERY (Linares, Geertsma, & Poolman, 2010) into pBADcLIC (Geertsma & Poolman, 2007) as *Nco*I*-Hin*dIII fragment. Plasmid pBADcLIC-GFP-ErmC was constructed in two steps. First, the GFP gene was amplified from pBADcLIC-GFP, using primer-introduced sites at the 5' (*Swa*I) and 3' (*Eco*RI*-Xba*I) ends; the gene was subsequently cloned as a *Swa*I*-Xba*I fragment in the pBADcLIC vector, yielding pBADcLIC-GFP*Eco*RI (the *Eco*RI site being created by the reverse primer). A *Eco*RI-XbaI fragment, corresponding to the *ermC*-His sequence, amplified from pREcLIC-ErmC, was cloned into pBADc-LIC-GFP*Eco*RI, yielding pBADcLIC-GFP-ErmC.

### Target proteins

The target genes coding for BcaP (branched-chain amino acid permease from *Lactococcus lactis*), LacSΔIIA (a C-terminal truncation mutant of the lactose transporter from *Streptococcus thermophilus),* PacL (putative P-type cation transporter from *L. lactis*), YidC (preprotein translocase from *L. lactis*), GerAC (spore germination factor from *Bacillus subtilis*), OxaA (YidC-like protein from *L. lactis*), GltP (glutamate transporter from *E. coli*) and Sav1866 (Multidrug ABC transporter from *Staphylococcus aureus*) were ligated into pBADcLIC-GFP-ErmC, using the ligation-independent cloning method (Geertsma & Poolman, 2007). In all cases, the target protein was expressed with a C-terminal tobacco etch virus protease cleavage site (sequence ENLYFQG) followed by the GFP, ErmC and a 10 Histag.

### Whole-cell fluorescence measurements

For whole-cell fluorescence measurements, cells expressing membrane protein-GFP-EmrC fusions were harvested, washed and subsequently resuspended to A₆₀₀=3 (1 mg/ml of total protein) in 100 mM KPi, pH 7. GFP emission was measured using a BioTek FL600 microplate fluorescence reader with the excitation wavelength set at 485 nm and the emission wavelength set at 530 nm (slit width of 20 and 25 nm, respectively). For direct comparison of the expression levels, the GFP fluorescence data were normalized to the same A₆₀₀. Background fluorescence levels were assessed by measuring whole-cell fluorescence of uninduced cells, and these values were subtracted.

### In-gel fluorescence and anti-his tag immune-detection

Whole-cell samples corresponding to 1 mg of total protein were resuspended in 400 µl ice-cold 50 mM KPi, pH 7.2, 1 mM MgSO₄, 10% glycerol, 1 mM PMSF plus trace amounts of DNase. Glass beads (300 mg, 0.1 mm diameter) were added and samples were shaken in a Tissue Lyser LT (Qiagen) at 50 oscillations/min for 5 min. Aliquots (10 µl, 25 µg protein) were taken and 2.5 µl of 5× protein sample buffer [120 mM Tris-HCl, pH 6.8, 50% (v/v) glycerol, 100 mM DTT, 2% (w/v) SDS plus 0.1% (w/v) bromophenol blue] was added. Samples were stored on ice until use. Protein samples were analyzed by 10% SDS-PAGE electrophoresis and in-gel GFP fluorescence, using a Fujifilm LAS-3000 imaging system and AIDA software (Raytest; Isotopenmessgeräte, GmbH). Subsequently, the gels were submitted to semi-dry electroblotting and immunodetection with a primary antibody raised against a hexa-His tag (Amersham Pharmacia Biotech). Chemiluminescence detection was done using the Western-Light kit (Tropix Inc.) and quantified using the Fujifilm LAS-3000 imaging system.

### Erythromycin resistance

The level of functional ErmC protein was estimated by quantification of bacterial survival as a function of erythromycin concentration. *E*. *coli* BW25113B cells expressing the different membrane protein-GFP-ErmC fusion were inoculated (2% v/v) into a fresh LB liquid medium with erythromycin concentrations varying from 0 to 100 µg/ml, and subsequently incubated overnight. The final optical density (A₆₀₀) reached by the culture was the parameter utilized to determine ErmC activity.

### Plasmid copy number and plasmid curing

The plasmid copy number was determined by isolation of plasmid DNA, using the Promega miniprep kit, and estimation of the plasmid DNA concentration by Nanodrop read-out at 260 nm. For curing of evolved strains of pBADc Lic-MP-GFP-ErmC, the cells were grown at 42°C in LB supplemented with 5µg/ml kanamycin and subsequent transfer (0.1 % v/v inoculum) into pre-warmed media. After 25 rounds of sub-culturing, the cells were plated on LB agar plates and isolated colonies were further characterized by culturing on LB agar plates supplemented with and without 100 µg/ml ampicillin. Single colonies of cured clones were purified further and the cells were retransformed with the original plasmids and screened for expression performance.

### Genome sequencing and data analysis

Genomic DNA was purified using the Qiagen Genomic DNA Purification Kit. Shotgun DNA libraries were generated according to the manufacturer's sample preparation protocol for genomic DNA. Briefly, 1-5 µg of genomic DNA was randomly sheared using nebulizers and the ends were repaired using polynucleotide kinase and Klenow enzyme. The 5' ends of the DNA fragments were phosphorylated and a single adenine base was added to the 3' ends using Klenow exo+. Following ligation of a pair of Illumina adaptors to the repaired ends, the DNA was amplified in 10 cycles, using adaptor primers (Illumina, San Diego, CA), and fragments around 250 bp long were isolated from agarose gels. Sequencing libraries were quantified with a Bio-Rad Experion Analyzer as well as the Picogreen fluorescence assay. Cluster generations were performed on an Illumina cluster station using 4 pmol of sequencing libraries. Seventy-six cycles of sequencing were carried out using the Illumina GenomeAnalyzerII system according to the manufacturer's specifications. Sequencing analysis was first done using the Illumina analysis pipeline. The output of the Illumina analysis pipeline was fed into CLC Bio Software (Aarhus,Denmark). At the assembly stage, sequence reads of each mutant were aligned to the previously assembled wildtype (reference) genome.

### RESULTS

### Choice of antibiotic resistance marker

The target (membrane) protein was fused to a C-terminal selectable marker that confers a drug resistance phenotype, when located in the cytoplasm. In the initial studies, we screened various antibiotic resistance markers, including the chloramphenicol (CAT), the tetracycline (TetA) and the kanamycin (Aph) resistance proteins as fusion partners for selection, but none of these markers proved useful in *E. coli.* Either the fusion partners were degraded or they dragged the target protein into a misfolded state. Because each of these antibiotic resistance proteins form an oligomeric complex (CAT and TetA are trimeric proteins and Aph is homodimeric), we reasoned that their quaternary structure and/or cellular location (TetA is a membrane protein) posed a problem, in particular when the membrane protein target would form an oligomer as well. We then identified the erythromycin-resistance protein (ErmC) as a monomeric, soluble antibiotic resistance protein (Linares et al, 2010). Fusions of membrane proteins with ErmC proved highly stable.

### Choice of expression host

The first requirement for the suitability of ErmC-fusions is a high sensitivity of the expression host for erythromycin. As many *E. coli* strains are relatively insensitive to erythromycin, we evaluated isogenic strains with different capacity to excrete antibiotics, that are *E. coli* BW25113, *E. coli* BW25113A (*ΔacrB*) and BW25113B (*ΔacrB ΔemrE mdfA*::kan) (Tal & Schuldiner, 2009). The differences in growth in erythromycin-containing media were marked. *E. coli* BW25113 could grow up to 100 µg/ml of erythromycin, albeit that the final yield was reduced. By comparison, *E. coli* BW25113B was completely inhibited at 10 µg/ml erythromycin, whereas *E. coli* BW25113A displayed an intermediate phenotype. *E. coli* BW25113B was selected as expression host for the (membrane) protein-GFP-ErmC fusions, hereafter referred to as MP-GFP-ErmC.

### The genetic selection system

Because ErmC can only be used as selection marker, we needed an additional protein partner to report functionality of the target protein. We thus constructed tandem fusions with GFP-ErmC C-terminal of the target protein. It has been established that GFP can serve as folding reporter and quantitatively assess the functionality of membrane proteins. In brief, when a protein, fused N-terminal to GFP, becomes misfolded during the biosynthesis, it turns GFP into a mis-folded, SDS-sensitive state. If the protein becomes properly folded, the GFP β-barrel will be assembled as a fluorescent SDS-resistant moiety; the SDS-sensitive and SDS-resistant conformations can be readily discriminated on SDS-PAGE and immunoblots. Whole-cell and in-gel fluorescence of protein extracts have been shown to match the activity of membrane proteins such as the lactose transporters from *Streptococcus thermophilus* (LacSΔIIA) and *E*. *coli* (LacY) and the glutamate transporter (GltP) from *E. coli* ((Drew et al., 2006; Geertsma et al., 2008).

MP-GFP-ErmC fusions were made with LacSΔIIA, BcaP, GltP, Sav1866, YidC and OxaA as target membrane protein. We observed two differently migrating protein species on immunoblots, the lower one being fluorescent in the in-gel assay (SFig. 2). These data are consistent with previous observations on single fusions and show that a fraction of the target protein is well-folded and another fraction is misfolded. Thus, GFP can be used as reporter of the folding state of membrane proteins in dual fusions with ErmC. We also made fusions of GFP-ErmC with the water-soluble proteins GerAC (spore germination factor) and LmoSBP (substrate-binding protein).

### Small-scale expression screening

To determine the optimal evolution screening conditions, we performed several small-scale expression studies. As variables we chose eight candidate membrane proteins, two *E. coli* hosts (MC1061 and BW25113), five temperatures (20, 25, 30, 37 and 42 °C) and various inducer (L-arabinose) concentrations. The expression of the fusion proteins was evaluated by whole cell and in-gel fluorescence measurements and anti-His immunoblots. SFig. 2 shows data from a representative set of experiments. For all the membrane proteins tested, the expression profiles were similar for the two *E. coli* strains (data not shown). The temperature at the time of production proved to be a key determinant for functional overexpression. The highest levels of functional expression (fluorescence) were generally associated with growth at relatively low temperature and either 20 or 25 °C being optimal. At 37 and 42 °C, hardly any fluorescence was observed and the fusion proteins were detected on immunoblots as a single band that is predicted to be misfolded.

A temperature 20 °C in combination with the strain BW25113B and an inducer concentration of 0.001% (w/v) L-arabinose was subsequently chosen as default condition to test the functionality of the MP-GFP-ErmC systems in a 96-well format, i.e. by comparing growth on selective media (with varying concentrations of erythromycin). In the presence of erythromycin without induction, none of the constructs supported growth. With 0.001% L-arabinose and in the presence of erythromycin, the cells expressing the target fusions survived. However, the final A₆₀₀ reached varied, consistent with the different levels of expression of the proteins. As shown in Fig. 1B and 1C, cells overexpressing GerAC reached the highest cell denstity, i.e. A₆₀₀ ∼0.9 at 50 µg/ml of erythromycin. Cells expressing GltP, YidC and OxaA reached an intermediate A₆₀₀ of ∼0.5, whereas poorly expressed membrane proteins like BcaP, LacSΔIIA and PacL supported growth up to A₆₀₀ of ∼0.2. Thus, our selection system effectively discriminates between cells expressing high levels of target membrane protein and those expressing little or no protein.

### Selection of improved expression hosts

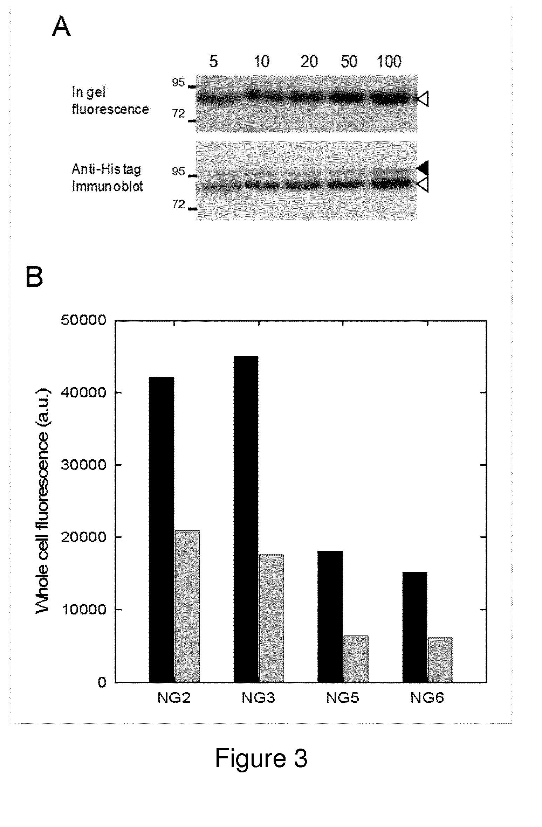

For the selection of BW25113B derivatives with improved expression properties, we used LacSΔIIA, GltP and BcaP as target proteins and thus constructed pBADc LacSΔIIA-GFP-ErmC, pBADc GltP-GFP-ErmC and pBADcBcaP-GFP-ErmC. LacSΔIIA and BcaP express poorly and GltP expresses relatively well in BW25113B (Fig. 1B). E. *coli* BW25113B bearing a plasmid for expression of the different MP-GFP-ErmC fusions was grown in LB plus ampicillin and kanamycin, and the concentration of erythromycin was gradually increased. Cells were transferred to fresh medium with an increasing amount of erythromycin every 48h. Cultures resistant to 100 µg/ml erythromycin were plated on LB agar with 100 µg/ml erythromycin to obtain individual clones, which were then screened for folded membrane protein by monitoring the GFP fluorescence in whole cells (Fig. 3A) and analyzing selected samples on SDS-PAGE to confirm full-length expression of MP-GFP-ErmC fusions. Finally, four clones (NG2, NG3, NG5 and NG6) showing increased erythromycin resistance and increased GFP fluorescence were selected for a more complete analysis.

### Plasmid characterization and curing

To exclude the possibility that the improved membrane protein expression relates to a mutation in the vector, *e.g.* causing altered plasmid copy, promoter activity or mutation in the target gene to be overexpressed, the plasmids were isolated from *E. coli* NG2, NG3, NG5 and NG6 and retransformed into *E. coli* BW25113B. The plasmid copy number was not altered and sequencing of the plasmids confirmed that the promoter region and genes were not mutated (Fig. 3B). Next, we cured *E*. *coli* NG2, NG3, NG5 and NG6 from its plasmids, and retransformed the evolved vector-free hosts with the parental plasmids. Testing of the expression levels of the fusion proteins in the evolved strains by whole cell and in-gel fluorescence and by immunoblotting confirmed that the alterations leading to enhanced protein production reside on the *E. coli* chromosome.

### Establishing optimal expression conditions

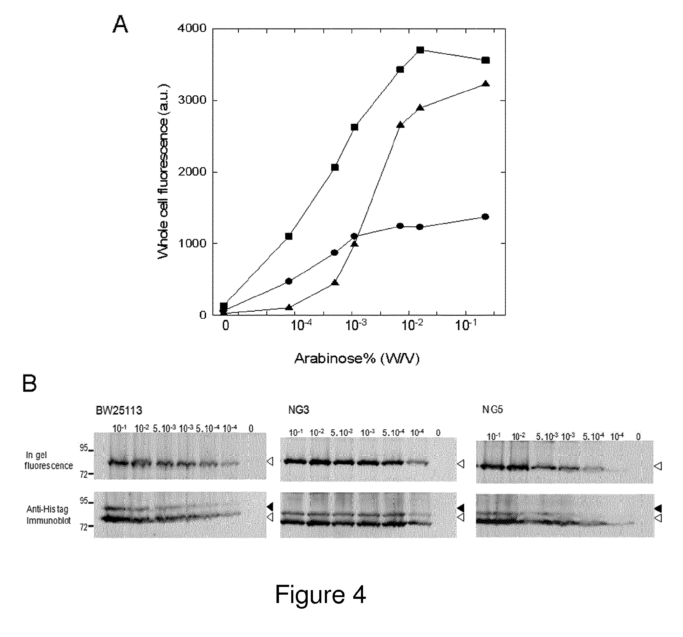

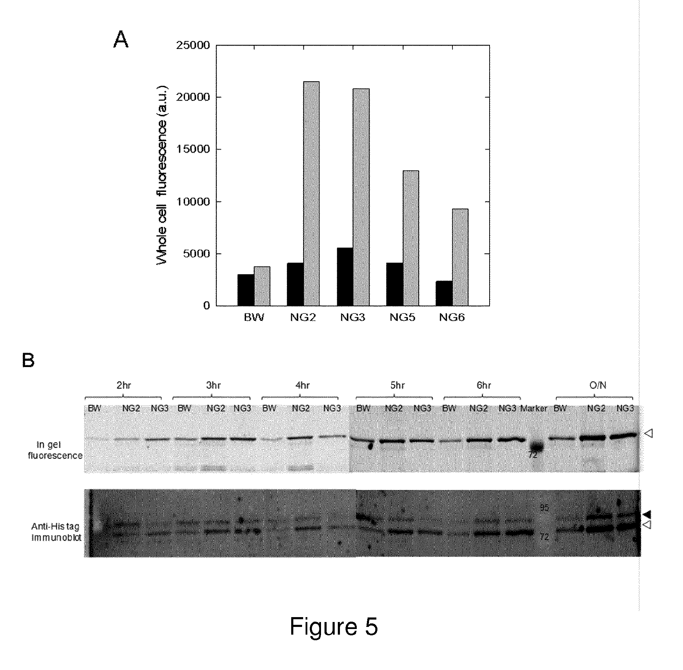

To determine the optimal expression conditions, we performed several small-scale expression studies. First, the optimal inducer, L-arabinose, concentration was determined. Fig. 4 shows the expression levels in the evolved and parental strains. NG3 and NG5 shows higher levels of expression compared to BW25113B but also the sensitivity for L-arabinose is shifted to lower concentrations. Second, we analyzed the induction kinetics at an L-arabinose concentration of 10⁻⁴ % (w/v) or higher. In case of NG3 and NG5, 10⁻² % (w/v) or higher concentrations of L-arabinose were required for maximal expression (Fig.4). We observed that the expression levels increase with induction times. The highest levels of protein were generally found after overnight induction but in most cases high levels were already obtained after 5-6 h (Fig. 5).

### Protein expression in the evolved E. coli strains

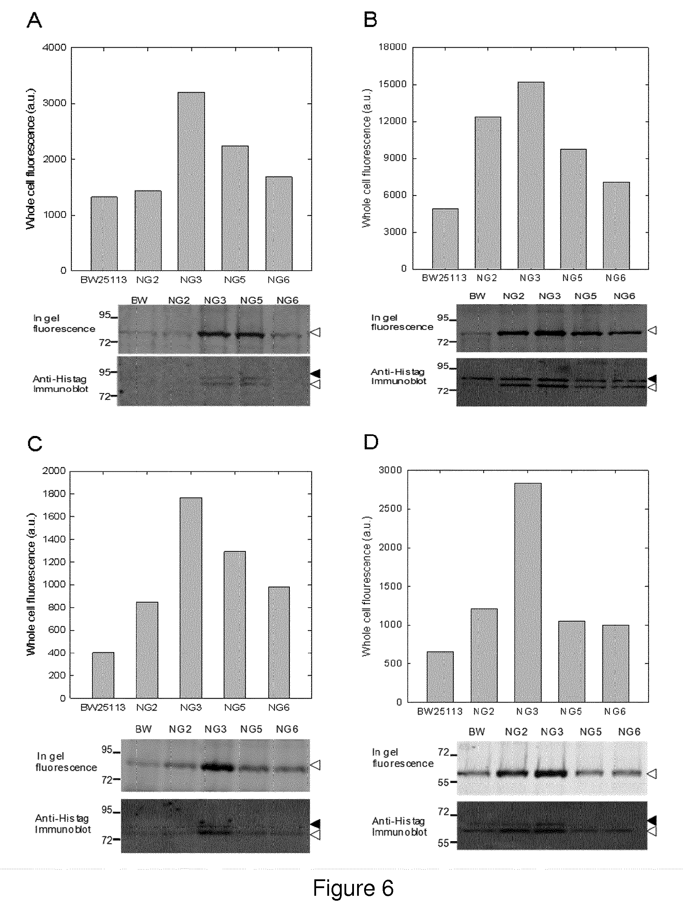

The cured NG strains were re-transformed with pBADcBcaP-GFP-ErmC and pBADc GltP-GFP-ErmC to confirm that the acquired property for enhanced expression is located on the *E. coli* chromosome and to check for differences in expression patterns. The expression levels and global folding of the proteins were tested by whole cell fluorescence (total GFP fluorescence), in-gel fluorescence (to discriminate between full length proteins and possible breakdown products), and anti-His tag immunoblots (to discriminate between folded and mis-folded fusion proteins (Geertsma et al., 2008). Fig. 6 shows that in each of the NG strains, the amount of fluorescent (folded) protein and the ratio of folded over mis-folded protein has increased relative to the parental strain BW25113B. Of the evolved strains, NG3 performed best overall, whereas NG2 did better in the overexpression GltP as compared to LacSΔIIA and BcaP. We note that NG2 was selected using GltP as target membrane protein, whereas NG5 and NG6 were obtained with BcaP. To test whether or not erythromycin is required for increased expression of the membrane protein-GFP-ErmC fusions, we performed expression trials with and without the antibiotic. Fig. 6 shows that the expression of LacSΔIIA did not require erythromycin. Finally, we tested the expression of two soluble reporters, that are GerAC and LmoSBP, and we observed that the level of GerAC was the same in BW25113B and the NG strains, whereas the production of Lmo SBP was significantly increased in the evolved *E. coli* strains.

### DNA sequencing of the evolved E. coli strains

Whole genome sequencing of the parent strain BW25113B and four evolved NG strains was done to explore the mechanism responsible for improved membrane protein production. The overall sequence coverage was more than 500-fold across the entire genome. The parent strain BW25113B was found to have 22 differences (mostly point mutations, 2 insertion mutations) relative to the sequence of E. coli K-12 sub-strain MG1655 (U00096.2). When compared to BW25113B, the evolved NG strains carried two or three mutations as summarized in Table 2. Remarkably, each of the NG strains had a mutation associated with *hns,* either in the promoter region or the structural gene. The *hns* mutation in NG5 and NG6 leads to a deletion of the amino acids Thr25-Glu28 at the dimerization interface of HNS. This deletion is predicted to destabilize dimer formation. Because dimerization (or higher order oligomerization) must precede DNA-binding, the mutation will lead to a loss of function of H-NS. The evolved *E. coli* NG2 and NG3 strains have a nonsense mutation in *hns.* In addition, NG2 has a mutation in the promoter region of *hns,* which most likely arose prior to the nonsensense and only caused partial loss of H-NS function Strikingly and in accordance with the genotype of the *hns* mutations, NG2 and NG3 yielded the highest overexpression of the membrane proteins. Table 1 summarizes the characterization of four *E. coli* strains, evolved towards improved membrane protein production. Without wishing to be bound by theory, it is possible that the secondary and/or tertiary mutations in the NG strains enhance the effects of the mutations in *hns.* It is also possible that the additional mutation(s) partially neutralize(s) the effect of *hns* inactivation such that the host cell remains viable.

**Table 1. Characterization of evolved E. coli strains**

| **Name** | **Genes** | **Coding region change** | **Amino acid change** | **Function of protein** |
|---|---|---|---|---|
| NG2 | *hns* | C376T | Gln126 to stop codon | Chromosome organization and transcriptional silencing |
| | *hns promoter* | | | Chromosome organization and transcriptional silencing |
| | *ung* | G220A | Gly74Ser | Uracil DNA glycosylase that modulates the mutation frequency; controlled by the *cpx* regulon |
| | *cpxA* | G524A | Ser175Asn | Histidine sensor kinase involved in cell envelope stress response |
| NG3 | *hns* | C376T | Gln126 to stop codon | Chromosome organization and transcriptional silencing |
| | *dcm* | T1337A | Val1446Glu | DNA-cytosine methyltransferase |
| NG5 | *hns* | Deletion A73 to A84 | Deletion Thr25, Leu26, Glu27 and Glu28 in HNS dimerization domain | Chromosome organization and transcriptional silencing |
| | *ydcU* | C348A | | Putative ABC transporter for spermidine and putrescine |
| | *yffN* | C195T | | Unknown |
| NG6 | *hns* | Deletion A73 to A84 | Deletion Thr25, Leu26, Glu27 and Glu28 in HNS dimerization domain | Chromosome organization and transcriptional silencing |
| | *YdcU* | C348A | | Putative ABC transporter for spermidine and putrescine |

### Construction of hns deletion strains

To confirm whether or not the *hns* mutation is responsible for the increased expression levels, the gene was deleted from the chromosome of *E. coli* BW25113B, MG1655 and MC1061. The qualitative results indicate that functional deletion of HNS increases the level of membrane protein expression.

### References

1. Arechaga, I., Miroux, B., Karrasch, S., Huijbregts, R., de Kruijff, B., Runswick, M. J., et al. (2000). FEBS Letters, 482(3), 215-219.
2. Bill, R. M., Henderson, P. J., Iwata, S., Kunji, E. R., Michel, H., Neutze, R., et al. (2011). Nature Biotechnology, 29(4), 335-340.
3. Bloch, V., Yang, Y., Margeat, E., Chavanieu, A., Auge, M. T., Robert, B., et al. (2003). Nature Structural Biology, 10(3), 212-218.
4. Bouvier, J., Gordia, S., Kampmann, G., Lange, R., Hengge-Aronis, R., & Gutierrez, C. (1998). Molecular Microbiology, 28(5), 971-980.
5. Casadaban, M. J., & Cohen, S. N. (1980). Journal of Molecular Biology, 138(2), 179-207.
6. Dame, R. T., Noom, M. C., & Wuite, G. J. (2006). Nature, 444(7117), 387-390.
7. Dar, M. E., & Bhagwat, A. S. (1993). Molecular Microbiology, 9(4), 823-833.
8. Dorman, C. J. (2004). Nature Reviews.Microbiology, 2(5), 391-400.
9. Drew, D., Lerch, M., Kunji, E., Slotboom, D. J., & de Gier, J. W. (2006). Nature Methods, 3(4), 303-313.
10. Drew, D., Newstead, S., Sonoda, Y., Kim, H., von Heijne, G., & Iwata, S. (2008). Nature Protocols, 3(5), 784-798.
11.Duguay, A. R., & Silhavy, T. J. (2004). Biochimica Et Biophysica Acta, 1694(1-3), 121-134.
12. Geertsma, E. R., Groeneveld, M., Slotboom, D. J., & Poolman, B. (2008). Proceedings of the National Academy of Sciences of the United States of America, 105(15), 5722-5727.
13. Geertsma, E. R., & Poolman, B. (2007). Nature Methods, 4(9), 705-707.
14. Georgiou, G., & Valax, P. (1996). Current Opinion in Biotechnology, 7(2), 190-197.
15. Grisshammer, R., & Tate, C. G. (1995). Quarterly Reviews of Biophysics, 28(3), 315-422.
16. Higgins, M. K., Demir, M., & Tate, C. G. (2003). Biochimica Et Biophysica Acta, 1610(1), 124-132.
17. Khnouf, R., Olivero, D., Jin, S., Coleman, M. A., & Fan, Z. H. (2010). Analytical Chemistry, 82(16), 7021-7026.
18. Klepsch, M. M., Persson, J. O., & de Gier, J. W. (2011). Journal of Molecular Biology, 407(4), 532-542.
19. Krogh, A., Larsson, B., von Heijne, G., & Sonnhammer, E. L. (2001). Journal of Molecular Biology, 305(3), 567-580.
20. Laible, P. D., Scott, H. N., Henry, L., & Hanson, D. K. (2004). Journal of Structural and Functional Genomics, 5(1-2), 167-172.
21. Lang, B., Blot, N., Bouffartigues, E., Buckle, M., Geertz, M., Gualerzi, C. O., et al. (2007). Nucleic Acids Research, 35(18), 6330-6337.
22. Linares, D. M., Geertsma, E. R., & Poolman, B. (2010). Journal of Molecular Biology, 401(1), 45-55.
23. Liu, Y., Chen, H., Kenney, L. J., & Yan, J. (2010). Genes & Development, 24(4), 339-344.
24. Loll, P. J. (2003). Journal of Structural Biology, 142(1), 144-153.
25. Lundstrom, K. (2004). Combinatorial Chemistry & High Throughput Screening, 7(5), 431-439.
26. Macintyre, G., Doiron, K. M., & Cupples, C. G. (1997). Journal of Bacteriology, 179(19), 6048-6052.
27. Marreddy, R. K., Geertsma, E. R., Permentier, H. P., Pinto, J. P., Kok, J., & Poolman, B. (2010). PloS One, 5(4), e10317.
28. Massey-Gendel, E., Zhao, A., Boulting, G., Kim, H. Y., Balamotis, M. A., Seligman, L. M., et al. (2009). Protein Science : A Publication of the Protein Society, 18(2), 372-383.
29. Maxwell, K. L., Mittermaier, A. K., Forman-Kay, J. D., & Davidson, A. R. (1999). Protein Science : A Publication of the Protein Society, 8(9), 1908-1911.
30. Mergulhao, F. J., Summers, D. K., & Monteiro, G. A. (2005). Biotechnology Advances, 23(3), 177-202.
31. Miroux, B., & Walker, J. E. (1996). Journal of Molecular Biology, 260(3), 289-298.
32. Newstead, S., Kim, H., von Heijne, G., Iwata, S., & Drew, D. (2007). Proceedings of the National Academy of Sciences of the United States of America, 104(35), 13936-13941.
33. Oberg, F., Ekvall, M., Nyblom, M., Backmark, A., Neutze, R., & Hedfalk, K. (2009). Molecular Membrane Biology, 26(4), 215-227.
34.Ogasawara, H., Teramoto, J., Hirao, K., Yamamoto, K., Ishihama, A., & Utsumi, R. (2004). Journal of Bacteriology, 186(24), 8317-8325.
35. Pogliano, J., Lynch, A. S., Belin, D., Lin, E. C., & Beckwith, J. (1997). Genes & Development, 11(9), 1169-1182.
36. Raman, P., Cherezov, V., & Caffrey, M. (2006). Cellular and Molecular Life Sciences : CMLS, 63(1), 36-51.
37. Schlegel, S., Klepsch, M., Gialama, D., Wickstrom, D., Slotboom, D. J., & de Gier, J. W. (2010). Microbial Biotechnology, 3(4), 403-411.
38. Sette, M., Spurio, R., Trotta, E., Brandizi, C., Brandi, A., Pon, C. L., et al. (2009). The Journal of Biological Chemistry, 284(44), 30453-30462.
39. Shaw, A. Z., & Miroux, B. (2003). Methods in Molecular Biology (Clifton, N.J.), 228, 23-35.
40.Sohail, A., Lieb, M., Dar, M., & Bhagwat, A. S. (1990). Journal of Bacteriology, 172(8), 4214-4221.
41. Steen, A., Wiederhold, E., Gandhi, T., Breitling, R., & Slotboom, D. J. (2011). Molecular & Cellular Proteomics : MCP, 10(7), M000052MCP200.
42. Tal, N., & Schuldiner, S. (2009). Proceedings of the National Academy of Sciences of the United States of America, 106(22), 9051-9056.
43. Tate, C. G., Whiteley, E., & Betenbaugh, M. J. (1999). The Journal of Biological Chemistry, 274(25), 17551-17558.
44. Wagner, S., Bader, M. L., Drew, D., & de Gier, J. W. (2006). Trends in Biotechnology, 24(8), 364-371.
45. Wagner, S., Klepsch, M. M., Schlegel, S., Appel, A., Draheim, R., Tarry, M., et al. (2008). Proceedings of the National Academy of Sciences of the United States of America, 105(38), 14371-14376.
46. Waldo, G. S., Standish, B. M., Berendzen, J., & Terwilliger, T. C. (1999). Nature Biotechnology, 17(7), 691-695.
47. Wang, D. N., Safferling, M., Lemieux, M. J., Griffith, H., Chen, Y., & Li, X. D. (2003). Biochimica Et Biophysica Acta, 1610(1), 23-36.
48. Widmann, M., & Christen, P. (2000). The Journal of Biological Chemistry, 275(25), 18619-18622.
49. Yin, J., Li, G., Ren, X., & Herrler, G. (2007). Journal of Biotechnology, 127(3), 335-347.
50. Zhang, G., Hubalewska, M., & Ignatova, Z. (2009). Nature Structural & Molecular Biology, 16(3), 274-280.
51. Zimmerman, S. B. (2006). Journal of Structural Biology, 153(2), 160-175.
52. Zoonens, M., & Miroux, B. (2010). Methods in Molecular Biology (Clifton, N.J.), 601, 49-66*.* doi:10.1007/978-1-60761-344-2_4

## Claims

1. A method for enhanced expression of a recombinant protein of interest in a microbial host cell, comprising
- providing a microbial host cell wherein the function of H-NS (histone-like nucleoid structuring protein) or a homolog thereof is impaired;
- transforming the host cell with an expression vector comprising a nucleotide sequence encoding the recombinant protein; and
- culturing the transformed host cell in a culture media suitable for the expression of the recombinant protein.

2. Method according to claim 1, wherein providing the host cell comprises inactivating a gene comprising
a) the DNA sequence shown in positions 1 to 414 in SEQ ID NO 1;
b) a DNA sequence encoding an amino acid sequence shown in positions 1 to 137 in SEQ ID NO 2 or a homologous amino acid sequence, or
c) a DNA sequence which is at least 70%, preferably at least 80%, more preferably at least 90%, identical to the DNA sequence of item (a) or item (b).

3. Method according to claim 1 or 2, wherein the H-NS homolog is Hha or StpA.

4. Method according to any one of claims 1-3, wherein the host cell has at least one mutation in one or more genes selected from the group consisting of *ung, cpxA, dcm* and *ydcU,* and homologs thereof.

5. Method according to any of the preceding claims, further comprising subjecting the host cell to an analysis of at least one property of the recombinant protein of interest.

6. A method to identify a microbial host cell having an improved capacity to express a functional and properly folded protein of interest, preferably a membrane protein, the method comprising the steps of
(a) providing a population of microbial host cells, wherein the host cell is E. *coli* strain BW25113A (Δ*acrB*) or BW25113B (Δ*acrB ΔemrE ΔmdfA:kan*)transformed with an expression vector comprising a target nucleic acid sequence encoding the target protein, wherein the target nucleic acid is C-terminally fused in tandem to a folding reporter GFP construct and to a selectable marker encoding the 23S-rRNA adenine N-6-methyltransferase (ErmC) ;
(b) inducing expression of the proteins encoded by the expression vector
(c) serial culturing the transformed host cells at gradually increasing erythromycin concentrations;
(d) selecting the host cell(s) showing increased expression of the reporter construct and,
(e) optionally, analyzing the genotype of the selected host cell(s).

7. Method according to claim 6, wherein the protein folding reporter construct encodes a fluorescent protein, preferably GFP or related fluorescent protein.

8. Method according to claim 6 or 7, wherein the host cell is *E. coli* strain BW25113B.

9. Method according to any one of the preceding claims, wherein the protein of interest is a eukaryotic protein

10. Method according to any one of the preceding claims, wherein the protein of interest is a membrane protein.

11. Use of a microbial cell wherein the function of H-NS or a homolog thereof is impaired as host cell for expression of a recombinant protein, preferably a membrane protein.

12. A microbial host cell wherein the function of H-NS or a homolog thereof is impaired, the host cell comprising an expression vector with gene(s) encoding a (heterologous or homologous) recombinant protein of interest, preferably a membrane protein.

13. Host cell according to claim 12, wherein the host cell is an *E*. *coli* cell comprising an expression vector wherein the gene is under the control of an inducible E. *coli* operon.

14. Host cell according to claim 13, wherein the inducible operon is the L-arabinose operon containing the P_{BAD} promoter of the araBAD operon.
